# EUROPEAN PATENT SPECIFICATION

(11) **EP 4 599 881 B1**
(45) Date of publication and mention of the grant of the patent: **17.06.2026**
(21) Application number: 24175262.5
(22) Date of filing: 10.05.2024
(51) Int. Cl.: A61N 1/04, A61N 1/36

(54) **PANTS FOR REPAIRING PELVIC FLOOR MUSCLES**
HOSE ZUR REPARATUR DER BECKENBODENMUSKULATUR
PANTALON POUR RÉPARER DES MUSCLES DU PLANCHER PELVIEN

(30) Priority: 06.02.2024 CN 202410171634
(43) Date of publication of application: 13.08.2025
(73) Proprietor: Xiamen Solex High-Tech Industries Co., Ltd., Xiamen, Fujian 361028 (CN)
(72) Inventor: WANG, Lixia, Xiamen, 361028 (CN); HONG, Chunjie, Xiamen, 361028 (CN)
(74) Representative: Verscht, Thomas Kurt Albert

(56) References cited:
- WO-A1-2023/179877
- AU-A1- 2015 384 447
- CA-A1- 2 945 677
- US-A1- 2016 303 363
- US-A1- 2020 360 681

## Description

### FIELD OF THE DISCLOSURE

The present disclosure relates to pants for repairing pelvic floor muscles.

### BACKGROUND OF THE DISCLOSURE

Pelvic floor dysfunction diseases in females are a series of diseases of pelvic organ prolapse caused by dysfunction of the pelvic floor support structure due to various factors. As China enters an aging society, the pelvic floor dysfunction diseases are increasing year by year, seriously affecting life qualities of elderly female, and can easily cause mental and psychological problems. The diseases have gradually been taken into account, and pelvic floor muscle exercise is an emerging activity in the field of obstetrics and gynecology in recent years and acts as important driving forces in improving female pelvic floor function.

For females, the pelvic floor muscles support many organs in the pelvic cavity, such as the bladder, uterus, small intestine, rectum, and normal functions of these organs are maintained through a contraction of a group of the pelvic floor muscles. The pelvic floor muscles have powerful functions, but are easily injured, resulting in females suffering from the pelvic floor muscle dysfunction diseases. The function of the pelvic floor muscles will be affected by an increase in intra-abdominal pressure caused by pregnancy, childbirth, surgery, coughing, etc., resulting in incontinence, uterine prolapse, decreasing quality of sexual life, etc. Therefore, females need to perform rehabilitation trainings of the pelvic floor muscles to protect the function of the pelvic floor muscles after childbirth.

Some solutions using smart pants for repairing the pelvic floor muscles have been emerged on the market. Electrodes in the pants for repairs are disposed on inner thighs, repairing positions are inaccurately positioned so as to act on the inner thighs or other locations without pelvic floor muscles, and repair effects are poor. In some other pants for repairs, positive and negative conductive fabrics are arranged left and right, so that fabrics are easily wrinkled by personal behaviors due to the fabrics on the crotch area being too much and too wide, resulting in a product failure caused by contact short-circuits of the positive and negative electrodes.

With regard to the prior art attention is drawn to WO 2023/179877 A1 from which a gear for electromyostimulation of pelvic floor musculature through a perineum of a human body is known. The gear includes a positioning support, and a first contact electrode coupled at a predefined position with the positioning support. The positioning support is shaped to be worn on the human body and configured to position the first contact electrode on the perineum of the human body. The gear includes a first conductor connected to the first contact electrode, and an electrical connector comprising a first electrical terminal. The first conductor is connected the first electrical terminal. The electrical connector is configured for connecting to an electromyostimulation current generator that generates an electrical stimulation current to be applied by the first contact electrode to the perineum for the electromyostimulation of the pelvic floor musculature.

Further, from CA 2 945 677 A1 a skin surface electrode is known, which is used in the treatment of incontinence. The electrode has one or more conductive regions and an egress through which bodily fluid may pass without substantially adversely affecting tissue contact in the conductive regions of the electrode. The electrode is configured for placement proximate a patient's perineal tissues. An incontinence treatment system includes an electrode, wearable signal generator and interface module.

Furthermore, from US 2016/303363 A1 apparatuses, methods, and systems for simulating low and/or high intensity exercise are known. More particularly, this document relates to an exercise mimetic device for simulating low and/or high intensity exercise using low intensity electrical stimulation to generate low intensity muscle contractions such as a wearable garment that preferably imitates exercise by eliciting low grade muscle contractions in several of the larger skeletal muscle groups in the body. The apparatus of various embodiments is a neuromuscular electrostimulation (NMES) device/garment with a control unit that is wirelessly connected to and controls a stimulator unit that generates and transmits a low intensity electrical stimulation within certain unique parameters. In various embodiments, the NMES device/garment is for treating conditions including but not limited to obesity, obesity related conditions such as diabetes, muscle toning, and/or other conditions benefitted by exercise. In various embodiments, the NMES device/garment is an over the counter (OTC) NMES device/garment.

Moreover, from US 2020/360681 A1 a conductive circuit is known, which may be applied to a garment, for example a garment for the stimulation of pelvic floor muscles. In the illustrated embodiments in the garment comprises a pair of shorts. The shorts are made from stretch fabric comprising a polyamide and are designed to fit closely to the body. The shorts are provided with two legs and a waistband area. The shorts comprise left and right conductive circuits, each of which comprises one or more electrodes formed from a printed conductive layer.

Finally, from AU 2015 384 447 A1 a muscle electrical stimulation device is known, which is provided with a main body, a power source part accommodated in the main body, an electrode part to which electric power is supplied from the power source part, a control part for controlling the supplying of electric power to the electrode part, and an operating part for changing a control mode of the control part. The electrode part is provided with a first electrode group and a second electrode group. The first electrode group extends from the main body so as to be positioned on a right-hand side of a person relative to a center line of the main body. The second electrode group extends from the main body so as to be positioned on a left-hand side of the person relative to the center line when the device is attached to an abdominal region. The device is configured so that electricity can be conducted between the first electrode group and the second electrode group via the person, and the first electrode group and the second electrode group combined include four or more electrodes.

### BRIEF SUMMARY OF THE DISCLOSURE

The present invention relates to pants for repairing pelvic floor muscles as defined in claim 1.

Preferred embodiments are disclosed in the dependent claims.

An objective of the present disclosure is to overcome the deficiencies in the existing techniques and provides pants for repairing pelvic floor muscles.

In order to solve the aforementioned technical problem, the present disclosure provides pants for repairing pelvic floor muscles, it comprises a wearing pants body and an electrical stimulation member disposed on the wearing pants body; the electrical stimulation member is disposed on a crotch position of the wearing pants body; and
the electrical stimulation member comprises a first flexible electrode and a second flexible electrode, and the first flexible electrode and the second flexible electrode respectively correspond to front and rear sides of the pelvic floor muscles of a user to apply electrical stimulation to the pelvic floor muscles, wherein the crotch position of the wearing pants body is divided into a front section and a rear section, the first flexible electrode is disposed on the front section, and the second flexible electrode is disposed on the rear section, and wherein the first flexible electrode at least partially extends into the rear section, and the second flexible electrode at least partially extends into the front section.

In a preferred embodiment, the first flexible electrode covers the front section, and the second flexible electrode covers the rear section.

In a preferred embodiment, the crotch position of the wearing pants body further comprises a stay-away section, the first flexible electrode and the second flexible electrode stay away from the stay-away section, and the stay-away section corresponds to clitoris and anus of the user.

In a preferred embodiment, the first flexible electrode and the second flexible electrode are detachably disposed on the wearing pants body.

In a preferred embodiment, the first flexible electrode and the second flexible electrode are respectively connected to the wearing pants body by magnetic attraction or glue.

In a preferred embodiment, the wearing pants body comprises a first annular connecting member and a second annular connecting member, the first annular connecting member is connected to the first flexible electrode, and the second annular connecting member is connected to the second flexible electrode.

In a preferred embodiment, the electrical stimulation member further comprises a controller disposed on the wearing pants body, the controller is configured to produce microcurrent stimulation on the pelvic floor muscles by the first flexible electrode and the second flexible electrode, the electrical stimulation member further comprises a first electrical connecting member and a second electrical connecting member, the first electrical connecting member and the second electrical connecting member are connected to the controller, the first flexible electrode is detachably connected to the first electrical connecting member, and the second flexible electrode is detachably connected to the second electrical connecting member.

In a preferred embodiment, the first flexible electrode and the first electrical connecting member are connected through a metal four-piece buckle, and the second flexible electrode and the second electrical connecting member are connected through a metal four-piece buckle.

In a preferred embodiment, the first electrical connecting member and the second electrical connecting member are connected to the controller through a conductive coating layer printed on the wearing pants body; or

the first electrical connecting member and the second electrical connecting member are connected to the controller through wires or flexible conductive silicone.

In a preferred embodiment, the controller is detachably connected to the wearing pants body.

In a preferred embodiment, the first flexible electrode and the second flexible electrode are made of silver-fiber conductive cloth.

In a preferred embodiment, the first flexible electrode and the second flexible electrode are made of a conductive coating layer printed on the wearing pants body; or the first electrical connecting member and the second electrical connecting member are connected to a controller by flexible conductive silicone.

In a preferred embodiment, the first flexible electrode and the second flexible electrode form a plurality of transverse grooves separately disposed along a front and rear direction.

Compared with the existing techniques, the technical solution of the present disclosure has the following advantages.

The first flexible electrode and the second flexible electrode respectively correspond to the front and rear sides of the pelvic floor muscles of the user to apply the electrical stimulation to the pelvic floor muscles. Compared with wearable devices on the market that provide two electrodes corresponding to a right side and a left side of the pelvic floor muscles, the pants for repairing the pelvic floor muscles in the present disclosure provide the first flexible electrode and the second flexible electrode disposed on the front and rear sides of the crotch position, and a failure caused by contact between positive and negative electrodes will not occur. At the same time, this design can accurately position at a position of the pelvic floor muscles for targeted repair, a treatment area is large, a repairing range is wide, and a repairing effect is good. The pelvic floor muscles are long-strip muscles extending in a front and rear direction, and compared with arranging the two electrodes on left and right sides of the crotch position, in which a repairing range of the two electrodes on the left and right sides (i.e., a length of current pathway) is smaller, the first flexible electrode and the second flexible electrode are disposed on the front and rear sides of the crotch position in the present disclosure, and the repairing range is wider and the repairing effect is better.

The crotch position of the wearing pants body further comprises the stay-away section, and the first flexible electrode and the second flexible electrode stay away from the stay-away section; and the stay-away section corresponds to clitoris and anus of the user. The arrangement of the stay-away section can prevent the clitoris and anus of the user from current stimulation.

### BRIEF DESCRIPTION OF THE DRAWINGS

FIG. 1 is a diagrammatic view of a crotch position of a wearing pants body of pants for repairing pelvic floor muscles in a preferred embodiment of the present disclosure.
FIG. 2A is a diagrammatic view of a maximum working distance of the pants for repairing the pelvic floor muscles in the preferred embodiment of the present disclosure.
FIG. 2B is a diagrammatic view of a maximum working distance of the pants for repairing pelvic floor muscles in the existing techniques.
FIG. 3 is a diagrammatic view of disassembly and assembly between the wearing pants body of the pants for repairing the pelvic floor muscles and a first flexible electrode and a second flexible electrode in the preferred embodiment of the present disclosure.
FIG. 4 is a diagrammatic view of an action between the first flexible electrode or the second flexible electrode and the pelvic floor muscles in the preferred embodiment of the present disclosure.
FIG. 5 is a diagrammatic view of the first flexible electrode partially extending into a rear section and the second flexible electrode partially extending into a front section to define a staggered structure in the present disclosure.

### DETAILED DESCRIPTION OF THE EMBODIMENTS

The present disclosure will be further described below in combination with the accompanying drawings and specific embodiments.

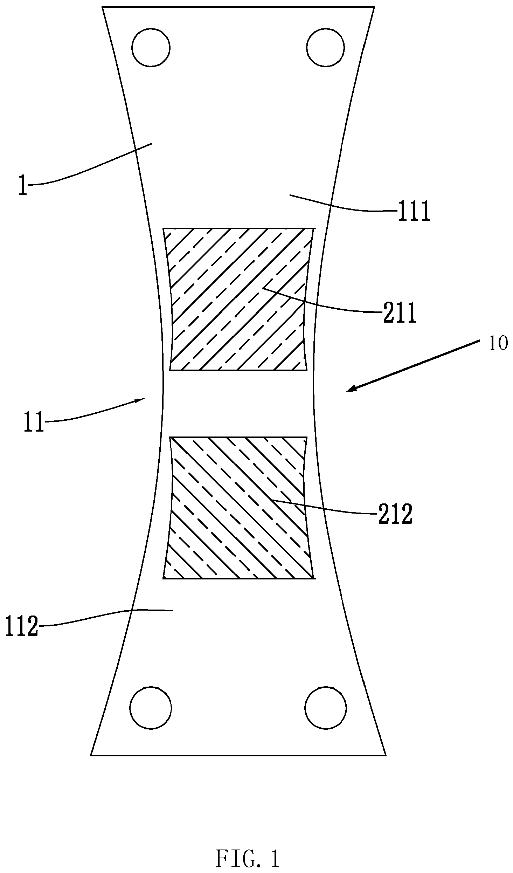

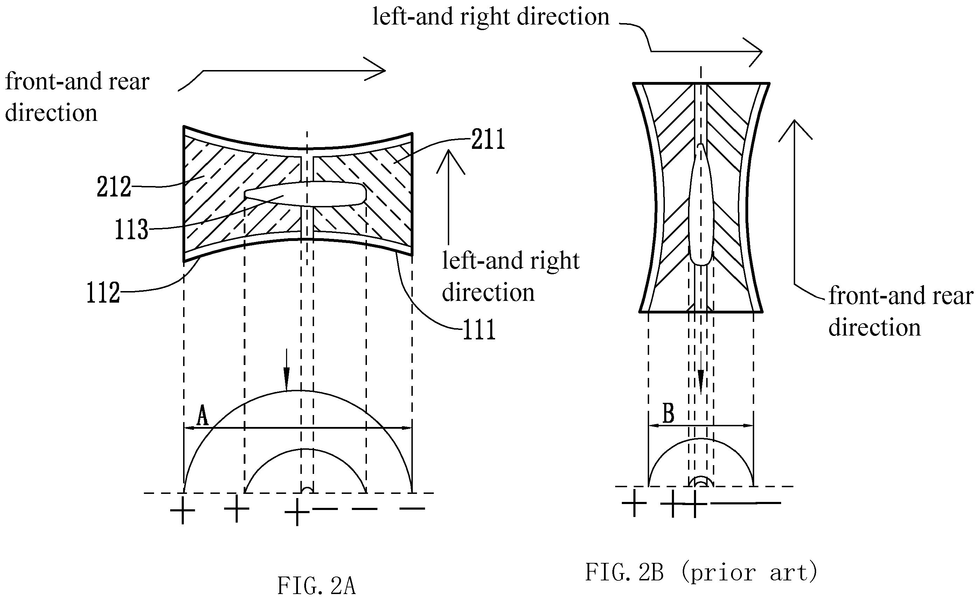

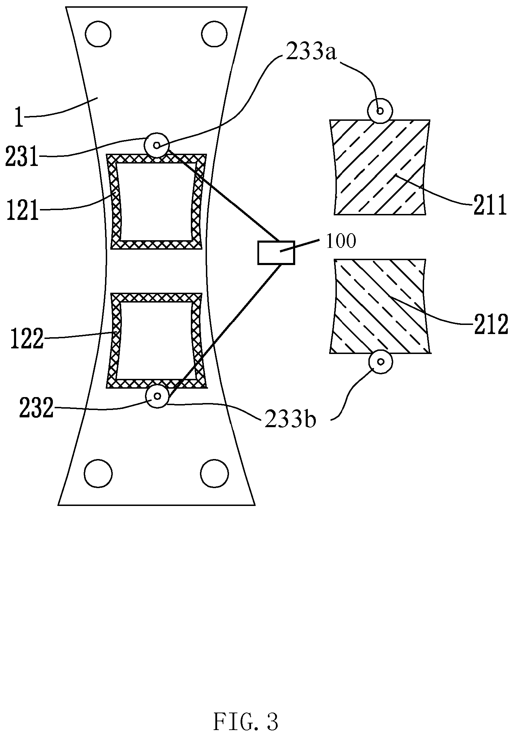

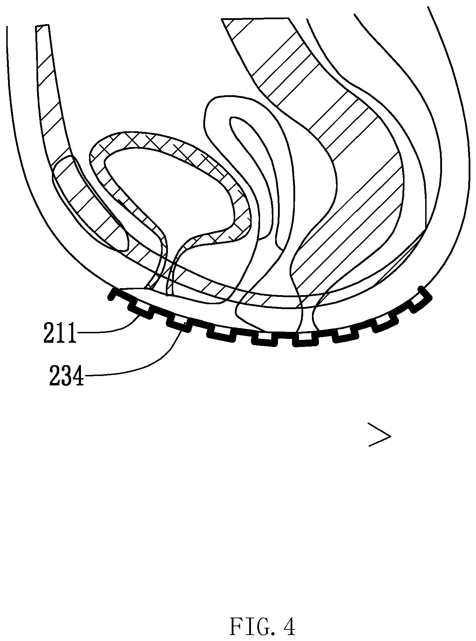

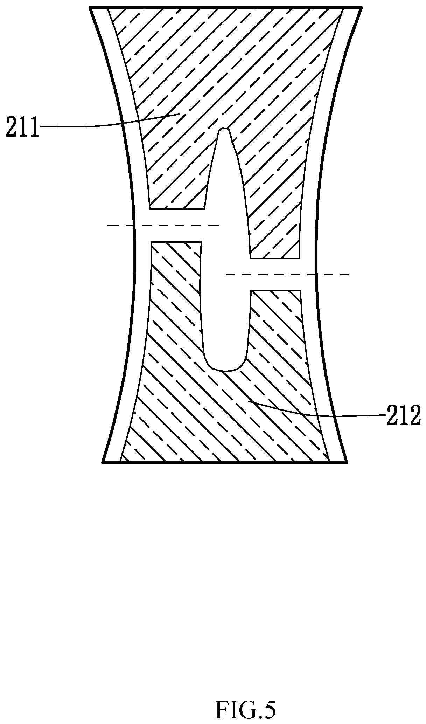

Referring to FIGS. 1-5, pants for repairing pelvic floor muscles comprise a wearing pants body 1 and an electrical stimulation member 10 disposed on the wearing pants body 1, the electrical stimulation member 10 is disposed on a crotch position 11 of the wearing pants body 1 corresponding to a perineum of a user. The crotch position 11 can be understood as a connection place of two pants legs, a top to a bottom of a middle part of the connection place can also be understood as a section of pants corresponding to thigh root and two-thigh intersection, i.e., a part where thighs are connected to a trunk. The electrical stimulation member 10 comprises a first flexible electrode 211 and a second flexible electrode 212, and the first flexible electrode 211 and the second flexible electrode 212 respectively correspond to a front side and a rear side of the pelvic floor muscles of the user to apply electrical stimulation to the pelvic floor muscles. The wearing pants body 1 may be in a shape of boxer briefs, etc., which is convenient to wear for the user when going out. Compared with wearable devices on the market that provide two electrodes corresponding to a right side and a left side of the pelvic floor muscles, the pants for repairing the pelvic floor muscles in the present disclosure provides the first flexible electrode 211 and the second flexible electrode 212 disposed on the front and rear sides of the crotch position 11, and a failure caused by contact between positive and negative electrodes will not occur. At the same time, this design can accurately position at a position of the pelvic floor muscles for targeted repairs, a treatment area is large, a repairing range is wide, and repairing effect is good. The pelvic floor muscles are long-strip muscles extending in a front and rear direction, and compared with arranging the two electrodes on left and right sides of the crotch position 11, in which a repairing range of the two electrodes on the left and right sides (i.e., a length of current pathway) is smaller, the first flexible electrode 211 and the second flexible electrode 212 are disposed on the front and rear sides of the crotch position 11 in the present disclosure, and the repairing range is wider and the repairing effect is better. Take a maximum working distance of positive and negative electrodes under microcurrent as an example: referring to FIG. 2A, in the present disclosure, a maximum working distance of a front and rear electrode placement is A; and in the existing techniques, referring to FIG. 2B, a maximum working distance of a left and right electrode placement is B. As shown in FIGS. 2A and 2B, three points, maximum, minimum, and middle distances under the microcurrent are respectively selected, A is much larger than B, it can be seen that the repairing range of the front and rear electrode placement is wider, an effective area applied to the pelvic floor muscles is also larger, and the repairing effect is better.

In this embodiment, the crotch position 11 of the wearing pants body 1 is divided into a front section 111 and a rear section 112, the first flexible electrode 211 is disposed on the front section 111, and the second flexible electrode is disposed on the rear section 112. The front section and the rear section 112 are separated by a preset distance in the front and rear direction using a transverse midline of the crotch position 11 as a boundary.

In this embodiment, the first flexible electrode 211 covers the front section 111, and the second flexible electrode 212 covers the rear section 112. In some simple replacements, the first flexible electrode 211 at least partially extends into the rear section 112, and the second flexible electrode 212 at least partially extends into the front section 111 to define a staggered structure.

In some simple replacements, the crotch position 11 of the wearing pants body 1 further comprises a stay-away section 113, and the first flexible electrode 211 and the second flexible electrode 212 stay away from the stay-away section 113; and the stay-away section 113 corresponds to clitoris and anus of the user. An arrangement of the stay-away section 113 can prevent the clitoris and anus of the user from current stimulation.

In this embodiment, the first flexible electrode 211 and the second flexible electrode 212 are detachably disposed on the wearing pants body 1. The first flexible electrode 211 and the second flexible electrode 212 are respectively connected to the wearing pants body 1 by magnetic attraction or glue. Specifically, the wearing pants body 1 comprises a first annular connecting member 121 and a second annular connecting member 122, the first annular connecting member 121 is connected to the first flexible electrode 211, and the second annular connecting member 122 is connected to the second flexible electrode 212. The first annular connecting member 121 and the second annular connecting member 122 may be an annular rubber magnet sewn by a sewing process for the magnetic attraction, and edges of the first flexible electrode 211 and the second flexible electrode 212 have magnets or ferromagnetic materials. Alternatively, the first annular connecting member 121 and the second annular connecting member 122 are annular double-sided tape for fixing the first flexible electrode 211 and the second flexible electrode 212 by bonding.

The electrical stimulation member 10 further comprises a controller 100 disposed on the wearing pants body 1, and the controller 100 is used to produce microcurrent stimulation on the pelvic floor muscles by the first flexible electrode 211 and the second flexible electrode 212; the electrical stimulation member 10 further comprises a first electrical connecting member 231 and a second electrical connecting member 232, the first electrical connecting member 231 and the second electrical connecting member 232 are connected to the controller 100, the first flexible electrode 211 is detachably connected to the first electrical connecting member 231, and the second flexible electrode 212 is detachably connected to the second electrical connecting member 232. Specifically, the first flexible electrode 211 and the first electrical connecting member 231 are connected through a metal four-piece buckle 233a, and the second flexible electrode 212 and the second electrical connecting member 232 are connected through a metal four-piece buckle 233b. The first flexible electrode 211 and the second flexible electrode 212 can be easily disassembled and assembled on the crotch position 11.

In this embodiment, the first electrical connecting member 231 and the second electrical connecting member 232 are connected to the controller 100 through a conductive coating layer printed on the wearing pants body 1, and the conductive coating layer can prevent discomfort caused by wires protruding from a surface of underwear. In some alternatives, the first electrical connecting member 231 and the second electrical connecting member 232 are connected to the controller 100 through wires or flexible conductive silicone.

In this embodiment, the controller 100 is detachably connected to the wearing pants body 1, and assembly-disassembly methods of the controller 100 can be attached to an outside of the wearing pants body 1 by hook and loop, etc.

In this embodiment, the first flexible electrode 211 and the second flexible electrode 212 are made of silver-fiber conductive cloth, or the first flexible electrode 211 and the second flexible electrode 212 are made of the flexible conductive silicone. The first flexible electrode 211 and the second flexible electrode 212 form a plurality of transverse grooves 234 separately disposed along the front and rear direction. A cloth of the wearing pants body 1 is ultra-fine nylon or other skin-friendly fabrics.

In some alternative embodiments, the first flexible electrode 211 and the second flexible electrode 212 are made of a conductive coating layer printed on the wearing pants body; or the first electrical connecting member 231 and the second electrical connecting member 232 are connected to the controller 100 by flexible conductive silicone.

The advantages of the transverse grooves 234 are as follows: 1. Accurate fit: the first flexible electrode 211 and the second flexible electrode 212 are formed by long strips using an embossing process, is elastic and close-fitting, has a staggered structure having a rough feeling, enables the fabric to be easily bent and deformed following a shape of the pelvic floor muscles, has a given sense of support, can replace a role of supporting cotton, etc., can achieve precise fit on skin, cause no stinging sensation stimulated by stronger local microcurrent, is breathable and comfortable, can provide the most effective and comfortable repairing experience for the user, and provides a very good experience. 2. Effective water locking: ① Material: The silver-fiber conductive cloth usually consists of silver fibers and base materials, and the silver fibers have excellent conductivity and water absorption properties. ② Shape: a main function of the long-strip rough design is to increase a contact area and a frictional force, when water is sprayed on the conductive cloth, water molecules contact the grooves, due to a special shape and a rough surface thereof, the water molecules are adsorbed in the grooves, more water will be stored and be not easily volatilized due to a temperature being lower compared with a flat surface, so that water is effectively locked. ③ Viscosity force: A surface of the long-strip rough structure has viscosity properties, i.e., the surface has an adhesion effect on liquid, and the viscosity force can resist flow of the liquid, resulting in forming certain retention of the liquid on the surface of the structure. ④ Capillarity effect: it refers to the flow of the liquid in small pipes caused by surface tension, in the long-strip rough structure, the liquid forms a series of small passages on the surface of the structure due to tiny grooves and protrusions on the surface of the structure, so that the capillarity effect is produced. ⑤ Self-attractive force: it refers to an attractive force of an object generated due to gravity, in the long-strip rough structure, the liquid forms a certain gravity distribution on the surface of the structure due to the tiny grooves and protrusions on the surface of the structure, thus producing the self-attractive force.

The disclosure may be summarized as follows: The present disclosure discloses pants for repairing pelvic floor muscles, it comprises a wearing pants body and an electrical stimulation member disposed on the wearing pants body; the electrical stimulation member is disposed on a crotch position of the wearing pants body corresponding to a perineum of a user; and the electrical stimulation member comprises a first flexible electrode and a second flexible electrode, and the first flexible electrode and the second flexible electrode respectively correspond to front and rear sides of the pelvic floor muscles of a user to apply electrical stimulation to the pelvic floor muscles. The technical solution provides pants for repairing pelvic floor muscles in which the repairing range is wider and the repairing effect is better.

## Claims

1. Pants for repairing pelvic floor muscles, the pants comprising a wearing pants body (1) and an electrical stimulation member (10) disposed on the wearing pants body; the electrical stimulation member (10) is disposed on a crotch position (11) of the wearing pants body (1); and
the electrical stimulation member (10) comprises a first flexible electrode (211) and a second flexible electrode (212), and the first flexible electrode (211) and the second flexible electrode (212) respectively correspond to front and rear sides of the pelvic floor muscles of a user to apply electrical stimulation to the pelvic floor muscles, wherein the crotch position (11) of the wearing pants body (1) is divided into a front section (111) and a rear section (112), the first flexible electrode (211) is disposed on the front section (111), and the second flexible electrode (212) is disposed on the rear section (112), and wherein the first flexible electrode (211) at least partially extends into the rear section (112), and the second flexible electrode (212) at least partially extends into the front section (111).

2. The pants for repairing the pelvic floor muscles according to claim 1, **characterized in that**: the first flexible electrode (211) covers the front section (111), and the second flexible electrode (212) covers the rear section (112).

3. The pants for repairing the pelvic floor muscles according to any one or more of claims 1 to 2, **characterized in that**: the crotch position (11) of the wearing pants body (1) further comprises a stay-away section (113), the first flexible electrode (211) and the second flexible electrode (212) stay away from the stay-away section (113), and the stay-away section (113) corresponds to clitoris and anus of the user.

4. The pants for repairing the pelvic floor muscles according to any one or more of claims 1 to 3, **characterized in that**: the first flexible electrode (211) and the second flexible electrode (212) are detachably disposed on the wearing pants body (1).

5. The pants for repairing the pelvic floor muscles according to any one or more of claims 1 to 4, **characterized in that**: the first flexible electrode (211) and the second flexible electrode (212) are respectively connected to the wearing pants body (1) by magnetic attraction or glue.

6. The pants for repairing the pelvic floor muscles according to any one or more of claims 1 to 5, in particular, according to claim 4 or 5, **characterized in that**: the wearing pants body (1) comprises a first annular connecting member (121) and a second annular connecting member (122), the first annular connecting member (121) is connected to the first flexible electrode (211), and the second annular connecting member (122) is connected to the second flexible electrode (212).

7. The pants for repairing the pelvic floor muscles according to any one or more of claims 1 to 6, **characterized in that**: the electrical stimulation member (10) further comprises a controller (100) disposed on the wearing pants body (1), the controller (100) is configured to produce current stimulation on the pelvic floor muscles by the first flexible electrode (211) and the second flexible electrode (212), the electrical stimulation member (10) further comprises a first electrical connecting member (231) and a second electrical connecting member (232), the first electrical connecting member (231) and the second electrical connecting member (232) are connected to the controller (100), the first flexible electrode (211) is detachably connected to the first electrical connecting member (231), and the second flexible electrode (212) is detachably connected to the second electrical connecting member (232).

8. The pants for repairing the pelvic floor muscles according to claim 7, **characterized in that**: the first flexible electrode (211) and the first electrical connecting member (231) are connected through a metal four-piece buckle (233a), and the second flexible electrode (212) and the second electrical connecting member (232) are connected through a metal four-piece buckle (233b).

9. The pants for repairing the pelvic floor muscles according to claim 7 and/or 8, **characterized in that**: the first electrical connecting member (231) and the second electrical connecting member (232) are connected to the controller (100) through a conductive coating layer printed on the wearing pants body (1); or
the first electrical connecting member (231) and the second electrical connecting member (232) are connected to the controller (100) through wires or flexible conductive silicone.

10. The pants for repairing the pelvic floor muscles according to any one or more of claims 7 to 9, **characterized in that**: the controller (100) is detachably connected to the wearing pants body (1).

11. The pants for repairing the pelvic floor muscles according to any one or more of claims 1 to 10, **characterized in that**: the first flexible electrode (211) and the second flexible electrode (212) are made of silver-fiber conductive cloth.

12. The pants for repairing the pelvic floor muscles according to any one or more of claims 1 to 11, **characterized in that**:
the first flexible electrode (211) and the second flexible electrode (212) are made of a conductive coating layer printed on the wearing pants body (1); or
the first electrical connecting member (231) and the second electrical connecting member (232) are connected to a controller (100) by flexible conductive silicone.

13. The pants for repairing the pelvic floor muscles according to any one or more of claims 1 to 12, **characterized in that**: the first flexible electrode (211) and the second flexible electrode (212) form a plurality of transverse grooves (234) separately disposed along a front and rear direction.

## Patentansprüche

1. Unterhose zum Reparieren von Beckenbodenmuskeln, wobei die Unterhose einen Trageunterhosenkörper (1) und ein elektrisches Stimulationsglied (10), das an dem Trageunterhosenkörper (1) angeordnet ist, aufweist; das elektrische Stimulationsglied (10) ist an einer Schrittposition (11) des Trageunterhosenkörpers (1) angeordnet; und
das elektrische Stimulationsglied (10) weist eine erste flexible Elektrode (211) und eine zweite flexible Elektrode (212) auf, und die erste flexible Elektrode (211) und die zweite flexible Elektrode (212) entsprechen jeweils Vorder- und Rückseiten der Beckenbodenmuskeln eines Anwenders, um eine elektrische Stimulation auf die Beckenbodenmuskeln anzuwenden, wobei die Schrittposition (11) des Trageunterhosenkörpers (1) in einen vorderen Abschnitt (111) und einen hinteren Abschnitt (112) unterteilt ist, die erste flexible Elektrode (211) ist an dem vorderen Abschnitt (111) angeordnet, und die zweite flexible Elektrode (212) ist an dem hinteren Abschnitt (112) angeordnet, und wobei die erste flexible Elektrode (211) sich wenigstens teilweise in den hinteren Abschnitt (112) erstreckt, und die zweite flexible Elektrode (212) erstreckt sich wenigstens teilweise in den vorderen Abschnitt (111).

2. Die Unterhose zum Reparieren der Beckenbodenmuskeln nach Anspruch 1, **dadurch gekennzeichnet, dass** die erste flexible Elektrode (211) den vorderen Abschnitt (111) abdeckt, und die zweite flexible Elektrode (212) deckt den hinteren Abschnitt (112) ab.

3. Die Unterhose zum Reparieren der Beckenbodenmuskeln nach einem oder mehreren der Ansprüche 1 bis 2, **dadurch gekennzeichnet, dass**: die Schrittposition (11) des Trageunterhosenkörpers (1) ferner einen Fernhalteabschnitt (113) aufweist, die erste flexible Elektrode (211) und die zweite flexible Elektrode (212) halten sich Fernhalteabschnitt (113) fern, und der Fernhalteabschnitt (113) entspricht der Klitoris und dem Anus des Anwenders.

4. Die Unterhose zum Reparieren der Beckenbodenmuskeln nach einem oder mehreren der Ansprüche 1 bis 3, **dadurch gekennzeichnet, dass** die erste flexible Elektrode (211) und die zweite flexible Elektrode (212) abnehmbar an dem Trageunterhosenkörper (1) angeordnet sind.

5. Die Unterhose zum Reparieren der Beckenbodenmuskeln nach einem oder mehreren der Ansprüche 1 bis 4, **dadurch gekennzeichnet, dass** die erste flexible Elektrode (211) und die zweite flexible Elektrode (212) jeweils mit dem Trageunterhosenkörper (1) durch magnetische Anziehung oder Klebstoff verbunden sind.

6. Die Unterhose zum Reparieren der Beckenbodenmuskeln nach einem oder mehreren der Ansprüche 1 bis 5, insbesondere nach Anspruch 4 oder 5, **dadurch gekennzeichnet, dass**: der Trageunterhosenkörper (1) ein erstes ringförmiges Verbindungsglied (121) und ein zweites ringförmiges Verbindungsglied (122) aufweist, das erste ringförmige Verbindungsglied (121) ist mit der ersten flexiblen Elektrode (211) verbunden, und das zweite ringförmige Verbindungsglied (122) ist mit der zweiten flexiblen Elektrode (212) verbunden.

7. Die Unterhose zum Reparieren der Beckenbodenmuskeln nach einem oder mehreren der Ansprüche 1 bis 6, **dadurch gekennzeichnet, dass**: das elektrische Stimulationsglied (10) ferner eine Steuereinrichtung (100) aufweist, die an dem Trageunterhosenkörper (1) angeordnet ist, die Steuereinrichtung (100) ist konfiguriert, um durch die erste flexible Elektrode (211) und die zweite flexiblen Elektrode (212) eine Stromstimulation an den Beckenbodenmuskeln zu produzieren, das elektrische Stimulationsglied (10) weist ferner ein erstes elektrisches Verbindungsglied (231) und ein zweites elektrisches Verbindungsglied (232) auf, das erst elektrische Verbindungsglied (231) und das zweite elektrische Verbindungsglied (232) sind mit der Steuereinrichtung (100) verbunden, die erste flexible Elektrode (211) ist lösbar mit dem ersten elektrischen Verbindungsglied (231) verbunden, und die zweite flexible Elektrode (212) ist lösbar mit dem zweiten elektrischen Verbindungsglied (232) verbunden.

8. Die Unterhose zum Reparieren der Beckenbodenmuskeln nach Anspruch 7, **dadurch gekennzeichnet, dass**: die erste flexible Elektrode (211) und das erste elektrische Verbindungsglied (231) sind durch eine vierteilige Metallschnalle (233a) verbunden, und die zweite flexible Elektrode (212) und das zweite elektrische Verbindungsglied (232) sind durch eine vierteilige Metallschnalle (233b) verbunden.

9. Die Unterhose zum Reparieren der Beckenbodenmuskeln nach Anspruch 7 und/oder 8, **dadurch gekennzeichnet, dass**: das erste elektrische Verbindungsglied (231) und das zweite elektrische Verbindungsglied (232) sind durch eine auf den Trageunterhosenkörper (1) gedruckte leitfähige Beschichtungsschicht mit der Steuereinrichtung (100) verbunden; oder
das erste elektrische Verbindungsglied (231) und das zweite elektrische Verbindungsglied (232) sind durch Drähte oder flexibles leitfähiges Silikon mit der Steuereinrichtung (100) verbunden.

10. Die Unterhose zum Reparieren der Beckenbodenmuskeln nach einem oder mehreren der Ansprüche 7 bis 9, **dadurch gekennzeichnet, dass**: die Steuereinrichtung (100) ist lösbar mit dem Trageunterhosenkörper (1) verbunden.

11. Die Unterhose zum Reparieren der Beckenbodenmuskeln nach einem oder mehreren der Ansprüche 1 bis 10, **dadurch gekennzeichnet, dass**: die erste flexible Elektrode (211) und die zweite flexible Elektrode (212) sind aus silberfaserleitfähigem Stoff hergestellt.

12. Die Unterhose zum Reparieren der Beckenbodenmuskeln nach einem oder mehreren der Ansprüche 1 bis 11, **dadurch gekennzeichnet, dass**:
die erste flexible Elektrode (211) und die zweite flexible Elektrode (212) sind aus einer leitfähigen Beschichtungsschicht, die auf den Trageunterhosenkörper (1) gedruckt ist, hergestellt; oder
das erste elektrische Verbindungsglied (231) und das zweite elektrische Verbindungsglied (232) sind durch flexibles leitfähiges Silikon mit einer Steuereinrichtung (100) verbunden.

13. Die Unterhose zum Reparieren der Beckenbodenmuskeln nach einem oder mehreren der Ansprüche 1 bis 12, **dadurch gekennzeichnet, dass**: die erste flexible Elektrode (211) und die zweite flexible Elektrode (212) bilden eine Vielzahl von quer verlaufenden Nuten (234), die getrennt entlang einer Vorder- und Rückrichtung angeordnet sind.

## Revendications

1. Pantalon de réparation des muscles du plancher pelvien, le pantalon comprenant un corps de pantalon (1) apte à être porté et un organe de stimulation électrique (10) disposé sur le corps de pantalon apte à être porté ; l'organe de stimulation électrique (10) est disposé sur une position d'entrejambe (11) du corps de pantalon (1) apte à être porté ; et
l'organe de stimulation électrique (10) comprend une première électrode flexible (211) et une seconde électrode flexible (212), et la première électrode flexible (211) et la seconde électrode flexible (212) correspondent respectivement aux côtés avant et arrière des muscles du plancher pelvien d'un utilisateur pour appliquer une stimulation électrique aux muscles du plancher pelvien, dans lequel la position d'entrejambe (11) du corps de pantalon (1) apte à être porté est divisée en une section avant (111) et une section arrière (112), la première électrode flexible (211) est disposée sur la section avant (111), et la seconde électrode flexible (212) est disposée sur la section arrière (112), et dans lequel la première électrode flexible (211) s'étend au moins partiellement dans la section arrière (112), et la seconde électrode flexible (212) s'étend au moins partiellement dans la section avant (111).

2. Pantalon de réparation des muscles du plancher pelvien selon la revendication 1, **caractérisé en ce que** :
la première électrode flexible (211) recouvre la section avant (111), et la seconde électrode flexible (212) recouvre la section arrière (112).

3. Pantalon de réparation des muscles du plancher pelvien selon l'une ou plusieurs des revendications 1 à 2, **caractérisé en ce que** : la position d'entrejambe (11) du corps de pantalon (1) apte à être porté comprend en outre une section d'écartement (113), la première électrode flexible (211) et la seconde électrode flexible (212) étant espacées de la section d'écartement (113), et la section d'écartement (113) correspond au clitoris et à l'anus de l'utilisateur.

4. Pantalon de réparation des muscles du plancher pelvien selon l'une ou plusieurs des revendications 1 à 3, **caractérisé en ce que** : la première électrode flexible (211) et la seconde électrode flexible (212) sont disposées de manière détachable sur le corps de pantalon (1) apte à être porté.

5. Pantalon de réparation des muscles du plancher pelvien selon l'une ou plusieurs des revendications 1 à 4, **caractérisé en ce que** : la première électrode flexible (211) et la seconde électrode flexible (212) sont respectivement reliées au corps de pantalon (1) apte à être porté par attraction magnétique ou au moyen d'une colle.

6. Pantalon de réparation des muscles du plancher pelvien selon l'une ou plusieurs des revendications 1 à 5, en particulier, selon la revendication 4 ou 5, **caractérisé en ce que** : le corps de pantalon (1) apte à être porté comprend un premier organe de liaison annulaire (121) et un second organe de liaison annulaire (122), le premier organe de liaison annulaire (121) est relié à la première électrode flexible (211), et le second organe de liaison annulaire (122) est relié à la seconde électrode flexible (212).

7. Pantalon de réparation des muscles du plancher pelvien selon l'une ou plusieurs des revendications 1 à 6, **caractérisé en ce que** : l'organe de stimulation électrique (10) comprend en outre un dispositif de commande (100) disposé sur le corps de pantalon (1) apte à être porté, le dispositif de commande (100) est configuré pour produire une stimulation de courant sur les muscles du plancher pelvien par la première électrode flexible (211) et la seconde électrode flexible (212), l'organe de stimulation électrique (10) comprend en outre un premier organe de connexion électrique (231) et un second organe de connexion électrique (232), le premier organe de connexion électrique (231) et le second organe de connexion électrique (232) sont connectés au dispositif de commande (100), la première électrode flexible (211) est connectée de manière détachable au premier organe de connexion électrique (231), et la seconde électrode flexible (212) est connectée de manière détachable au second organe de connexion électrique (232).

8. Pantalon de réparation des muscles du plancher pelvien selon la revendication 7, **caractérisé en ce que** : la première électrode flexible (211) et le premier organe de liaison électrique (231) sont connectés par l'intermédiaire d'un bouton-pression métallique en quatre parties (233a), et la seconde électrode flexible (212) et le second organe de liaison électrique (232) sont connectés par l'intermédiaire d'un bouton-pression métallique en quatre parties (233b).

9. Pantalon de réparation des muscles du plancher pelvien selon la revendication 7 et/ou 8, **caractérisé en ce que** : le premier organe de liaison électrique (231) et le second organe de liaison électrique (232) sont connectés au dispositif de commande (100) par l'intermédiaire d'une couche de revêtement conducteur imprimée sur le corps de pantalon (1) apte à être porté ; ou
le premier organe de connexion électrique (231) et le second organe de connexion électrique (232) sont connectés au dispositif de commande (100) par l'intermédiaire de fils ou de silicone conducteur flexible.

10. Pantalon de réparation des muscles du plancher pelvien selon l'une ou plusieurs des revendications 7 à 9, **caractérisé en ce que** : le dispositif de commande (100) est relié de manière détachable au corps de pantalon (1) apte à être porté.

11. Pantalon de réparation des muscles du plancher pelvien selon l'une ou plusieurs des revendications 1 à 10, **caractérisé en ce que** : la première électrode flexible (211) et la seconde électrode flexible (212) sont faites d'un tissu conducteur en fibres d'argent.

12. Pantalon de réparation des muscles du plancher pelvien selon l'une ou plusieurs des revendications 1 à 11, **caractérisé en ce que** :
la première électrode flexible (211) et la seconde électrode flexible (212) sont faites d'une couche de revêtement conducteur imprimée sur le corps de pantalon (1) apte à être porté ; ou
le premier organe de connexion électrique (231) et le second organe de connexion électrique (232) sont connectés à un dispositif de commande (100) par un silicone conducteur flexible.

13. Pantalon de réparation des muscles du plancher pelvien selon l'une ou plusieurs des revendications 1 à 12, **caractérisé en ce que** : la première électrode flexible (211) et la seconde électrode flexible (212) forment une pluralité de rainures transversales (234) disposées séparément le long d'une direction avant et arrière.
